# EUROPÄISCHE PATENTSCHRIFT

(11) **EP 1 180 136 B1**
(45) Veröffentlichungstag und Bekanntmachung des Hinweises auf die Patenterteilung: **10.05.2006**
(21) Anmeldenummer: 00935027.3
(22) Anmeldetag: 16.05.2000
(51) Int. Cl.: C12N 1/00

(54) **VERWENDUNG VON SELBSTEMULGIERENDEN ÖLEN IN FERMENTATIONSVERFAHREN**
UTILIZATION OF SELF-EMULSIFYING OILS IN FERMENTATION PROCESSES
UTILISATION D'HUILES AUTO-EMULSIFIANTES DANS DES PROCEDES DE FERMENTATION

(30) Priorität: 25.05.1999 DE 19923760
(43) Veröffentlichungstag der Anmeldung: 20.02.2002
(73) Patentinhaber: Cognis Deutschland GmbH & Co. KG, 40789 Monheim (DE)
(72) Erfinder: MOLITOR, Jean-Pierre, D-40589 Düsseldorf (DE); DE HAUT, Christian, F-77310 Boissise-le-Roi (FR); ABRIBAT, Benoit, F-91490 Dannemois (FR); WEGENER, Matthias, D-40597 Düsseldorf (DE); ROGGE, Bent, D-40227 Düsseldorf (DE)
(86) Internationale Anmeldenummer: PCT/EP2000/004367
(87) Internationale Veröffentlichungsnummer: WO 2000/071673

(56) Entgegenhaltungen:
- WO-A-00/17294

## Beschreibung

Die vorliegende Erfindung betrifft die Verwendung von selbstemulgierenden Ölen in Fermentationsverfahren.

Bei der Synthese komplexer Naturstoffe oder sonstiger organischer Verbindungen, beispielsweise Antibiotika, werden zunehmend mikrobiologische Verfahren eingesetzt. Dabei handelt es sich um eine Stoffumwandlung unter anaeroben oder aeroben Bedingungen, bei der Mikroorganismen, insbesondere aber Bakterien oder Pilze beteiligt sind. Für derartige Verfahren werden in der Fachwelt verschiedene, nicht immer klar voneinander abgegrenzte Ausdrücke, wie *"Bioconversion", "Biotransformation"* oder *"Fermentation"* verwendet. Der letztere Ausdruck wird auch im Rahmen der vorliegenden Anmeldung für solche Verfahren verwendet, bei denen Mikroorganismen, vorzugsweise Bakterien, zur Umwandlung bzw. Synthese von chemischen Verbindungen verwendet werden.

Für die Entwicklung und Optimierung von Fermentationsprozessen ist insbesondere das Reaktionsmedium, in dem die mikrobiologische Umwandlung stattfindet, von Bedeutung. Das Reaktionsmedium, in aller Regel eine wäßrige Lösung oder Dispersion, beeinflußt insbesondere die Ausbeute und Effizienz des Verfahrens. Die Mikroorganismen benötigen als Nährstoffe Kohlenstoff, Stickstoff und bestimmte Spurenelemente in gebundener Form, z.B. Calcium, Eisen, Phosphor oder Zink, um eine erfolgreiche Metabolisierung zu den gewünschten Produkten möglich zu machen. Weiterhin muß regelmäßig ein bestimmter, meistens enger Temperatur- und pH-Bereich eingehalten werden. Zu weiteren Einzelheiten sei hier auf das Lehrbuch von W. *Crueger*/A*. Crueger,* Biotechnologie - Lehrbuch der angewandten Mikrobiologie, 2. Auflage 1984, R. Oldenbourg Verlag, verwiesen. Insbesondere Kapitel 5 dieses Werkes beschäftigt sich mit den Grundlagen der Fermentationstechnik. Diese Literaturstelle gehört daher auch ausdrücklich zur Offenbarung der vorliegenden Anmeldung. Als Nährstoffe für die Mikroorganismen werden neben energiereichen Zuckern und deren Derivaten in vielen Verfahren zusätzlich natürliche Fette und Öle, sowie Derivate dieser Stoffklassen, wie Glycerin, Glyceride, Fettsäuren oder Fettsäureester eingesetzt. Selbstverständlich dürfen die Kulturmedien keine Inhaltsstoffe aufweisen, die die Metabolisierung der Mikroorganismen negativ beeinflussen können.

Aus der DE 37 38 812 A1 ist beispielsweise ein mikrobielles Verfahren zur Herstellung von alpha-omega-Dicarbonsäuren bekannt, wobei Bakterien des Stamms *Candida tropicalis* Methyllaurat in die gewünschten Dicarbonsäuren umwandeln. Diese Umwandlung findet in einem wäßrigen Medium bei einem pH-Wert von 6,0 und einer Temperatur von 30 °C statt. Das Medium enthält, neben den Mikroorganismen als Energielieferant Glucose, weiterhin als Emulgator ethoxyliertes Sorbitanmonooleat, Hefeextrakt, Maisquellwasser sowie anorganische N- und P-Quellen. Dem Medium wird dann das Methyllaurat zudosiert. Der Schrift ist kein Hinweis auf den Emulsionstyp zu entnehmen, der sich im Fermenter ausbildet bzw. in dem das Methyllaurat der Fermentationsbrühe zudosiert wird. Aus der EP 0 535 939 A1 ist ein Verfahren zur Herstellung von omega-9-mehrfach ungesättigten Fettsäuren bekannt, wobei in einem wäßrigen Kulturmedium geeignete Mikroorganismen in Gegenwart von Zuckern als Energielieferanten und anorganischen oder organischen Stickstoffquellen, sowie in Gegenwart von Fettsäuremethylestern die gewünschten mehrfach ungesättigten Fettsäuren produzieren.

Es sind aber auch Verfahren bekannt, wo nur Fettstoffe der oben bezeichnete Art als Energielieferanten verwendet werden. Dies ist besonders von wirtschaftlichem Interesse, da derartige Fettstoffe in der Regel preiswerte sind als Zucker, Stärke und ähnliche Verbindungen. Park et al. beschreiben (Park et al., Journal of Fermentation and Bioengineering, Vol. 82, No. 2, 183-186, 1996) einen Fermentationsprozess zur Herstellung von Tylosin, bei dem Mikroorganismen des Stammes *Streptomyces fradiae* in einem wäßrigen Medium verwendet werden, wobei als einzige Kohlenstoffquelle Rapsöl in Ausgangsmengen von etwa 60 g/l enthalten ist.

Bei Fermentationsverfahren spielt außerdem der Sauerstoffgehalt im Medium bzw. der Fermentationsbrühe, eine entscheidende Rolle. Dabei kommt dem Sauerstoff bei aeroben Prozessen die Rolle eines Substrates zu. Entscheidend ist, ob ein für das jeweilige Verfahren ausreichender Sauerstoffübergang von der Gas- in die Flüssigphase, die die Mikroorganismen enthält, stattfinden kann. Ein wichtiger Parameter stellt die spezifische Ausstauschfläche dar, die in der Regel indirekt über den Sauerstoffübergangskoeffizienten k_{La} bestimmt wird (vergl. Literaturstelle *Crueger,* Kapitel 5, Seite 71 ff). Die Einstellung des optimalen Sauerstoffeintrags erfolgt typischerweise durch Rühren der Fermentationsbrühe, wobei der Sauerstoff bzw. die Luft mit der Flüssigkeit vermischt wird und so an den Grenzflächen der Gasaustausch stattfindet. Allerdings kann der erhebliche mechanische Energieeintrag durch starkes Rühren, wie Park et al. ausführen, auch Teile der Kultur zerstören, und so die Ausbeute des Verfahrens verringern. Die abgestorbenen Mikroorganismen werden außerdem selbst weiter abgebaut und können durch die gebildeten Abbauprodukte zu einer Vergiftung der Kultur führen, die eine wirtschaftliche Produktion unmöglich macht. Aus der Arbeit von Goma und Rols (G. Goma, J.L. Rols, Biotech. Let., Vol 13, No. 1, Seiten 7 bis 12, 1991) ist bekannt daß die Verwendung von Sojaöl in Fermentationsverfahren zur Herstellung von Antibiotika zu einer Verbesserung des Sauerstoffübergangskoeffizienten k_{La} führt, was bei gleichem Energieeintrag (Rühren) zu einem Anstieg der Ausbeute des Gesamtverfahrens führen kann.

Der vorliegenden Erfindung lag nun die Aufgabe zugrunde, Fermentationsverfahren so zu verbessern, daß einerseits preiswerte Kohlenstoffquellen eingesetzt werden können und andererseits eine ausreichende Versorgung der Mikroorganismen mit Sauerstoff gewährleistet ist, ohne daß eine unzulässig hohe mechanische Belastung der Mikroorganismen durch Rühren auftritt. Es sollte eine Weg gefunden werden, den mechanischen Energieeintrag bei Fermentationsverfahren zu minimieren ohne daß es zu einer Verringerung der Ausbeute kommt vorzugsweise soll eine Erhöhung der Ausbeute trotz verringertem Energieeintrag möglich sein.

Es wurde gefunden, daß die Verwendung von selbstemulgierenden Ölen die obige Aufgabe löst. In einer ersten Ausführungsform wird die Verwendung von ausgewählten Verbindungen aus der Gruppe der ethoxylierten Triglyceride pflanzlichen Ursprungs in Fermentationsverfahren beansprucht.

Bei selbstemulgierenden Verbindungen handelt es sich um Substanzen, die bei Mischung mit Wasser ohne Zusatz weiterer Emulgatoren eine stabile Emulsion mit Wasser ausbilden. Die selbstemulgierenden Öle werden daher der wäßrigen Fermentationsbrühe entweder direkt, oder in Form einer wäßrigen Emulsion zudosiert. In der Fermentationsbrühe selbst bilden sich dann Öl-in-Wasser-Emulsionen aus. Die Fermentationsbrühe enthält die Mikroorganismen sowie die Stickstoffquelle und Spurenelemente und ggf. weitere Hilfsstoffe, insbesondere Entschäumer. Als Stickstoffquellen kommen beispielsweise in Betracht: Pepton, Hefe- oder Malzextrakt, Maisquellwasser, Harnstoff oder Lecithine. Die Spurenelemente können in Form anorganischer Salze anwesend sein, beispielsweise Natrium- oder Kaliumnitrat, Ammoniumnitrat, Ammoniumsulfat, Eisensulfat etc. Die Öle emulgieren ohne weiteren Energieeintrag, so daß ein verstärktes Rühren der Fermentationsbrühe nicht mehr notwendig ist und sich der Scherstreß für die Mikroorganismen verringert, was zu erhöhten Ausbeuten führt. Die Einzelheiten der Fermentationsverfahren, insbesondere die Geschwindigkeit und Menge der zudosierten Emulsion bzw. des Öls, ergeben sich aus der Art der Mikroorganismen und des gewählten Fermentationsverfahrens und können vom Fachmann an die spezifischen Gegebenheiten angepaßt werden.

Geeignete selbtsemulgierende Öle sind native Öle pflanzlichen Ursprungs, die auf bekannte Weise mit Ethylenoxid umgesetzt wurden. Es handelt sich dabei im Wesentlichen um Triglyceridmischungen, wobei das Glycerin mit längerkettigen Fettsäuren jeweils vollständig verestert ist. Besonders geeignete pflanzliche Öle sind ausgewählt aus der Gruppe Erdnuß-, Kokos-, Lein-, Palm-, Oliven-, Palmkern-, Ricinus-, Raps-, Sesam-, Soja- und Sonnenblumenöl. Ganz besonders bevorzugt sind Raps-, Soja- und Sonnenblumenöl.

Erdnußöl enthält durchschnittlich (bezogen auf Fettsäure) 54 Gew.-% Ölsäure, 24 Gew.-Linolsäure, 1 Gew.-% Linolensäure, 1 Gew.-% Arachinsäure, 10 Gew.-% Palmitinsäure, sowie 4 Gew.-% Stearinsäure. Der Schmelzpunkt beträgt 2 bis 3 °C.
Leinöl enthält typischerweise 5 Gew.-% Palmitin-, 4 Gew.-% Stearin-, 22 Gew.-% Öl-, 17 Gew.-% Linol- und 52 Gew.-% Linolensäure. Die Iodzahl liegt im Bereich von 155 bis 205, Die Verseifungszahl ist 188 bis 196 und der Schmelzpunkt liegt bei etwa - 20 °C.
Kokosöl enthält an Fettsäuren etwa 0,2 bis 1 Gew.-% Hexan-, 5 bis 8 Gew.-% Octan-, 6 bis 9 Gew.-% Decan-, 45 bis 51 Gew.-% Laurin-, 16 bis 19 Gew.-% Myristin-, 9 bis 11 Gew.% Palmitin-, 2 bis 3 Gew.-% Stearin-, weniger als 0,5 Gew.-% Behen-, 8 bis 10 Gew.-% Öl- und bis 1 Gew.-% Linolsäure. Die Iodzahl liegt im Bereich von 7,5 bis 9,5 , die Verseifungszahl liegt bei 0,88 bis 0,90. Der Schmelzpunkt liegt bei 20 bis 23 °C.
Olivenöl enthält überwiegend Ölsäure (vergl. Lebensmittelchem. Gerichtl. Chem., 39, 112 bis 114, 1985). Palmöl enthält als Fettsäurekomponenten etwa 2 Gew.-% Myristin-, 42 Gew.-% Palmitin-, 5 Gew.-% Stearin-, 41 Gew.-% Öl-, 10 Gew.-% Linolsäure. Palmkemöl ist typischerweise in Bezug auf das Fettsäurespektrum wie folgt zusammengesetzt: 9 Gew.-% Capron/Capryl/Caprin-, 50 Gew.-% Laurin-, 15 Gew.-% Myristin-, 7 Gew.-% Palmitin-, 2 Gew.-% Stearin-, 15 Gew.-% Öl- und 1 Gew.-% Linolsäure.
Rapsöl enthält als Fettsäurekomponenten typischerweise etwa 48 Gew.-% Erucasäure, 15 Gew.-% Ölsäure, 14 Gew.-% Linolsäure, 8 Gew.-% Linolensäure, 5 Gew.-% Icosensäure, 3 Gew.-% Palmitinsäure, 2 Gew.-% Hexadecensäure und 1 Gew.-% Docosadiensäure. Rapsöl aus neuer Züchtung ist bezüglich der ungesättigten Anteile angereichert. Typische Fettsäureanteile sind hier Erucasäure 0,5 Gew.-%, Ölsäure 63 Gew.-%, Linolsäure 20 Gew.-%, Linolensäure 9 Gew.-%, Icosensäure 1 Gew.-%, Palmitinsäure 4 Gew.-%, Hexadecensäure 2 Gew.-% und Docosadiensäure 1 Gew.-%.
Ricinusöl besteht zu 80 bis 85 Gew.-% aus dem Glycerid der Ricinolsäure, daneben sind zu etwa 7 Gew.-% Glyceride der Öl-, zu 3 Gew.-% Glyceride der Linol- und zu etwa 2 Gew.-% die Glyceride der Palmitin- und der Stearinsäure enthalten.
Sojaöl enthält zu 55 bis 65 Gew.-% der Gesamtfettsäuren mehrfach ungesättigte Säuren, insbesondere Linol- und Linolensäure. Ähnlich ist die Situation beim Sonnenblumenöl , dessen typisches Fettsäurespektrum, bezogen auf Gesamtfettsäure wie folgt aussieht: ca. 1 Gew.-% Myristin-, 3 bis 10 Gew.-% Palmitin-, 14 bis 65 Gew.-% Öl- und 20 bis 75 Gew.-% Linolsäure.
Alle obigen Angaben über den Fettsäureanteile in den Triglyceriden sind bekanntermaßen abhängig von der Qualität der Rohstoffe und können daher zahlenmäßig schwanken.

Die Öle werden mit Ethylenoxid unter Druck, gegebenenfalls in Anwesenheit von sauren oder basischen Katalysatoren alkoxyliert, wobei solche Ethoxylate bevorzugt sind, die pro Mol Triglycerid 1 bis 10, vorzugsweise 1 bis 5 und insbesondere 1 bis 3 Mol Ethylenoxid enthalten. Besonders bevorzugt sind Ethoxylate auf Basis Soja-, Sonnenblume- und/oder Rapsöl. Besonders bevorzugt sind Rapsöl mit 3 und/oder 5 Mol Ethylenoxid pro Mol Triglycerid sowie Sojaöl, ethoxyliert mit 2 Mol Ethylenoxid. Die Öle können dabei in beliebigen Abmischungen untereinander oder in reiner Form verwendet werden. Sie werden entweder direkt oder in Form wäßriger Emulsionen verwendet. Im letzteren Fall enthalten die Emulsionen 99 bis 1 Gew.-% Wasser, und zwischen 1 und 99 Gew.-% der selbstemulgierenden Öle. Vorzugsweise werden Emulsionen verwendet, die 90 bis 99 Gew.-% der selbstemulgierenden Öle und 1 bis 10 Gew.-% Wasser enthalten. Es ist möglich, wenn auch nicht bevorzugt, neben den Ölen noch andere Hilfsstoffe, wie Entschäumer oder Emulgatoren mit zu verwenden. Im Sinne der vorliegenden technischen Lehre wird der Fachmann jedoch bestrebt sein, möglichst wenig Zusatzstoffe in den Fermentationsprozeß einzutragen.

Die selbsemulgierenden Öle können in Fermentationsprozessen aller Art eingesetzt werden. Dabei können alle dem Fachmann bekannten Verfahrensausgestaltungen, z.B. Batch- oder Fed-Batch sowie kontinuierliche Fermentation verwendet werden. Auch sind alle dem Fachmann bekannten Fermentersysteme einsetzbar. Zu den Einzelheiten siehe *Crueger,* Seiten 50 bis 70. Die Verwendung der Mikroemulsionen ist auch nicht auf bestimmte Mikroorganismen begrenzt, vielmehr lassen sich die Emulsionen zur Herstellung oder Umwandlung aller dem Fachmann durch Fermentation bekannten Verbindungen einsetzten. Neben den klassischen Fermentationsverfahren, die überwiegend zu Synthese von Antibiotika eingesetzt werden, (vergl. *Crueger,* Seiten 197 bis 242) eignen sich die beschriebenen Emulsionen aber auch zum Einsatz bei mikrobiellen Transformationen ("Bioconversion"), z.B. der Transformation von Steroiden und Sterinen, von Antibiotika und Pestiziden oder der Herstellung von Vitaminen (vergl. *Crueger,* Seiten 254 bis 273). Bevorzugt ist aber die Verwendung in Fermentationsprozessen zur Herstellung von Antibiotika, beispielsweise Chephalosporinen, Tylosin oder Erythromycin.

Die erfindungsgemäße Verwendung der ethoxylierten Triglyceride zeigt beispielsweise bei der Herstellung von Penicillin G und Cephalosporinen vorteilhafte Wirkung. Im Vergleich zum Einsatz von nicht-ethoxyliertem Sojaöl (jeweils 1 Gew.-%) führt die Verwendung von ethoxylierten Triglyceriden zu einer verbesserten Emulgierung der Fermentationsbrühe und einem gesteigerten Sauerstofftransfer. Somit kann die Rührgeschwindigkeit im Fermenter herabgesetzt werden, ohne daß es zu einem Ausbeuteverlust kommt.

## Patentansprüche

1. Verwendung von selbstemulgierenden Ölen, ausgewählt aus der Gruppe der ethoxylierten Triglyceride pflanzlichen Ursprungs in Fermentationsverfahren.

2. Verwendung nach Anspruch 1, **dadurch gekennzeichnet, daß** Triglyceride pflanzlichen Ursprungs verwendet werden, die pro Mol Triglycerid mit 1 bis 10, vorzugsweise 1 bis 5 und insbesondere 1 bis 3 Mol Ethylenoxid umgesetzt wurden.

3. Verwendung nach den Ansprüchen 1 oder 2, **dadurch gekennzeichnet, daß** die selbstemulgierenden Öle ausgewählt sind aus der Gruppe ethoxyliertes Raps-, Soja- und/oder Sonnenblumenöl.

4. Verwendung nach Anspruch 1 oder 2, **dadurch gekennzeichnet, daß** die Öle in Form einer wäßrigen Emulsion verwendet werden.

5. Verwendung nach Anspruch 3, **dadurch gekennzeichnet, daß** solche Emulsionen verwendet werden, die, bezogen auf die Emulsion 99 bis 1 Gew.-% Wasser und 1 bis 99 Gew.-% selbstemulgierende Öle enthalten.

## Claims

1. The use of self-emulsifying oils selected from the group of ethoxylated triglycerides of vegetable origin in fermentation processes.

2. The use claimed in claim 1, **characterized in that** triglycerides of vegetable origin reacted with 1 to 10, preferably 1 to 5 and more particularly 1 to 3 moles ethylene oxide per mole triglyceride are used.

3. The use claimed in claim 1 or 2, **characterized in that** the self-emulsifying oils are selected from the group consisting of ethoxylated rapeseed oil, soybean oil and/or sunflower oil.

4. The use claimed in claim 1 or 2, **characterized in that** the oils are used in the form of an aqueous emulsion.

5. The use claimed in claim 3, **characterized in that** emulsions containing 99 to 1% by weight water and 1 to 99% by weight self-emulsifying oils, based on the emulsion, are used.

## Revendications

1. Utilisation d'huiles auto-émulsifiantes, choisies dans le groupe des triglycérides éthoxylés d'origine végétale dans des procédés de fermentation.

2. Utilisation selon la revendication 1,
**caractérisée en ce qu'**
on utilise des triglycérides d'origine végétale ayant réagi, pour chaque mole de triglycéride, avec 1 à 10, de préférence 1 à 5, et en particulier 1 à 3 moles d'oxyde d'éthylène.

3. Utilisation selon les revendications 1 ou 2,
**caractérisée en ce que**
les huiles auto-émulsifiantes sont choisies dans le groupe constitué d'huile de colza, de soja et/ou de tournesol éthoxylée.

4. Utilisation selon la revendication 1 ou 2,
**caractérisée en ce que**
les huiles sont utilisées sous la forme d'une émulsion aqueuse.

5. Utilisation selon la revendication 3,
**caractérisée en ce qu'**
on utilise des émulsions qui contiennent, par rapport à l'émulsion, 99 à 1 % en poids d'eau et 1 à 99 % en poids d'huiles auto-émulsifiantes.
